# EUROPEAN PATENT APPLICATION

(11) **EP 3 222 731 A1**
(43) Date of publication of application: **27.09.2017**
(21) Application number: 16161566.1
(22) Date of filing: 22.03.2016
(51) Int. Cl.: C12P 7/06

(54) **A METHOD OF PRODUCING ETHANOL**

(71) Applicant: Shell Internationale Research Maatschappij B.V., 2596 HR Den Haag (NL)
(72) Inventor: SPRACHMANN, Gerald, 1031HW Amsterdam (NL); GEERLINGS, Jacobus Johannes, 1031HW Amsterdam (NL)
(74) Representative: Matthezing, Robert Maarten

(57) **Abstract**

The present invention provides a method of producing ethanol, the method at least comprising the steps of:
(a) providing a CO₂-containing stream (10);
(b) converting at least a part of the CO₂ of the CO₂-containing stream (10) provided in step (a), thereby obtaining a CO-enriched stream (20);
(c) subjecting the CO-enriched stream (20,20') obtained in step (b) to bacterial fermentation, thereby obtaining at least an ethanol-enriched liquid stream (30) and a CO₂-enriched gaseous stream (40).

## Description

The present invention relates to a method of producing ethanol. In particular the present invention relates to a method of producing ethanol from a CO₂-containing stream. Several methods for producing ethanol are known in the art.

There has been a continuous desire in developing alternative processes that can convert CO₂ into valuable products, if only for reducing CO₂ emissions.

It is an object of the present invention to provide an alternative method for producing ethanol.

It is a further object of the present invention to provide a method of producing ethanol starting from a CO₂-containing stream.

One or more of the above or other objects can be achieved by providing a method of producing ethanol, the method at least comprising the steps of:
(a) providing a CO₂-containing stream;
(b) converting at least a part of the CO₂ of the CO₂-containing stream provided in step (a), thereby obtaining a CO-enriched stream;
(c) subjecting the CO-enriched stream obtained in step (b) to bacterial fermentation, thereby obtaining at least an ethanol-enriched liquid stream and a CO₂-enriched gaseous stream.

It has surprisingly been found according to the present invention that ethanol can be produced starting from a CO₂-containing stream, by using a combination of CO₂ reduction and bacterial fermentation.

In step (a), a CO₂-containing stream is provided. The person skilled in the art will readily understand that this CO₂-containing stream is not particularly limited and may have various origins, including waste streams. An example of a suitable CO₂-containing stream is an off-gas stream coming from Heavy Paraffin Synthesis.

Preferably, the CO₂-containing stream provided in step (a) comprises at least 5.0 vol.% CO₂, preferably at least 10.0 vol.%. Typically, the CO₂-containing stream provided in step (a) comprises at most 60 vol.% CO₂.

Typically, the CO₂-containing stream provided in step (a) comprises also some CO. Preferably, the CO₂-containing stream provided in step (a) comprises at least 2.0 vol.% CO, preferably at least 5.0 vol.%, more preferably at least 10.0 vol.% and typically at most 35 vol.%. Also, the CO₂-containing stream provided in step (a) typically comprises some H₂, such as at least 1.0 vol.% H₂, preferably at least 2.0 vol.%, more preferably at least 5.0 vol.%. Preferably, the CO₂-containing stream provided in step (a) typically comprises no O₂ or only very small amounts of O₂, such as below 0.2 vol.% O₂, preferably less than 0.1 vol.%, more preferably less than 0.05 vol.% or even less than 0.01 vol.%.

According to an especially preferred embodiment according to the present invention, the CO₂-containing stream provided in step (a) has been obtained from a preceding (or 'second') CO₂-containing stream that has been subjected to a preceding (or 'second') bacterial fermentation step thereby obtaining a second ethanol-enriched liquid stream. If this preceding CO₂-containing stream is referred to as 'second CO₂-containing stream', the CO₂-containing stream provided in step (a) can be referred to as 'first CO₂-containing stream'. Preferably, the preceding (or 'second') CO₂-containing stream comprises at least 10.0 vol.% CO₂, preferably at least 20 vol.% and typically at most 60 vol.%.

In step (b), at least a part of the CO₂ of the CO₂-containing stream provided in step (a) is converted, thereby obtaining a CO-enriched stream. The person skilled in the art will readily understand that the conversion of the CO₂ to obtain CO can be performed in many ways. As the person skilled in the art is familiar with such conversions, this is not discussed here in detail. Examples of suitable conversions include RWGS (Reverse Water Gas Shift) reactions, electrolysis or other CO₂ reduction process.

Preferably, the conversion in step (b) is performed in the presence of hydrogen (H₂). Preferably, at the start of the conversion, H₂ is present in an amount of from 20 to 60 vol.%. Preferably, the H₂/CO₂ volume ratio of the CO₂-containing stream provided in step (a) directly before it is converted in step (b) is at least 1.0, preferably at least 2.0, more preferably at least 4.0 and typically below 10.

Preferably, the conversion in step (b) is by a Reverse Gas Shift reaction.

According to an alternative embodiment according to the present invention, the conversion in step (b) is by electrolysis. As electrolysis is known per se, this is not further discussed here in detail. Preferably, the electrolysis is performed using Electrolytic-Based Fuel Cells.

Typically, the pressure during the conversion in step (b) is at least 2.0 bara, preferably at least 10 bara, more preferably at least 20 bara, even more preferably at least 40 bara and typically below 100 bara.

In step (c), the CO-enriched stream obtained in step (b) is subjected to bacterial fermentation, thereby obtaining at least an ethanol-enriched liquid stream and a CO₂-enriched gaseous stream.

The bacterial fermentation is not particularly limited and may be performed in various ways. As the person skilled in the art is familiar with bacterial fermentation, this is not discussed here in detail. Examples of suitable bacterial fermentations include gas fermentation processes as available from LanzaTech (Skokie, Illinois (USA)), Ineos (Rolle, Switzerland) and Coskata (Warrenville, Illinois (USA)). Preferably the bacterial fermentation is performed by at least one member of the Carboxydotrophic bacteria such as Clostridium autoethanogenum.

Typically, the bacterial fermentation of step (c) takes place at pressure of between 1 and 100 bara and temperatures of 5 to 100°C. Preferably, the bacterial fermentation in step (c) takes place at pressure of at least 2.0 bara, preferably at least 10 bara, more preferably at least 20 bara, even more preferably at least 40 bara. In case a preceding (or 'second') bacterial fermentation is to be used, then the same pressure and temperature ranges would typically be selected (although the actual conditions of the first and second bacterial fermentation may differ).

The person skilled in the art will readily understand that the ethanol-enriched liquid stream and CO₂-enriched gaseous stream obtained in step (c) can be further processed if needed. Typically, the liquid ethanol-enriched stream will be distilled, preferably together with the second ethanol-enriched liquid stream (if any). Thereafter, the distilled ethanol stream may be used as such, blended with other streams or further reacted to other products.

Preferably, the CO-enriched stream obtained in step (b) is separated to obtain a H₂-enriched stream (preferably containing from 50 to 99 vol.% H₂). This separation can for example be performed in a pressure-swing adsorption (PSA) device or using a H₂-selective membrane. According to an especially preferred embodiment according to the present invention, at least a part of the H₂-enriched stream is reused in the conversion in step (b). To this end, the H₂-enriched stream may directly be sent to the reactor in which the conversion of step (b) takes place. Alternatively, the H₂-enriched stream may first be combined with the CO₂-containing stream provided in step (a). As in some embodiments the H₂ is consumed, a H₂-makeup stream may be required. Preferably, such a H₂-makeup stream is produced using renewable power resources (biomass, H₂O hydrolysis using renewable power).

Hereinafter the invention will be further illustrated by the following non-limiting drawing. Herein shows:
Fig. 1 schematically a first embodiment of a process block scheme of a method according to the present invention;
Fig. 2 schematically a second embodiment of a process block scheme of a method according to the present invention, wherein two bacterial fermentations take place;
Fig. 3 schematically a third embodiment of a process block scheme of a method according to the present invention, wherein electrolysis is used; and
Fig. 4 schematically a fourth embodiment of a process block scheme of a method according to the present invention, wherein a H₂-separation step occurs before bacterial fermentation.

Same reference numbers refer to the same or similar components.

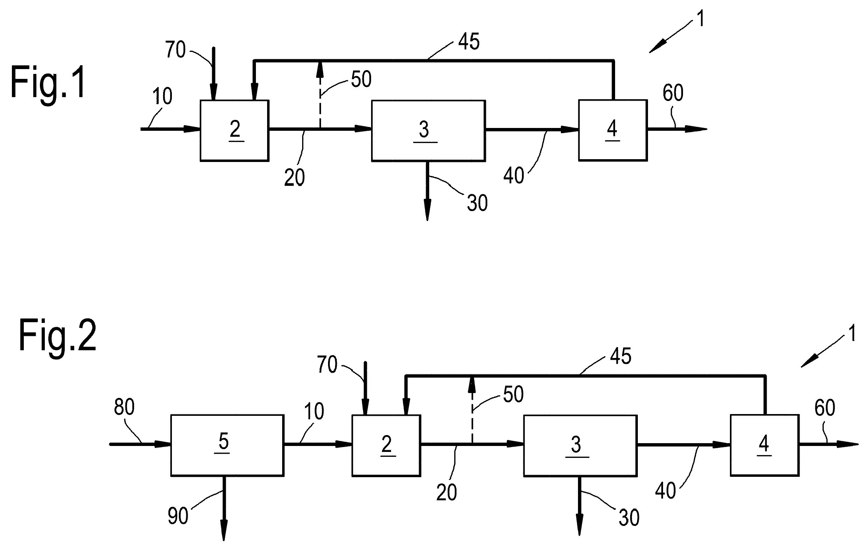

Figure 1 shows a process block scheme, generally referred to with reference number 1, for a method of producing ethanol. The process block scheme shows a first reactor 2 for converting CO₂ into CO (in the embodiment of Figure 1, using a Reverse Water Gas Shift reaction), a second reactor 3 (for performing bacterial fermentation) and a separator 4 (such as a Pressure-Swing Adsorption device) for removing H₂.

During use of the process of Fig. 1, a CO₂-containing stream 10 is provided (e.g. obtained from a Heavy Paraffin Synthesis process) and sent to the RWGS reactor 2. In the RWGS reactor 2 a CO-enriched stream 20 is obtained. In the embodiment of Fig. 1, a H₂ recycle stream 50 (obtained from the separator 4) and a H₂ make-up stream 70 are used to supply H₂ to the RWGS reactor 2.

The CO-enriched stream 20 is subjected in reactor 3 to bacterial fermentation, thereby obtaining an ethanol-enriched liquid stream 30 and a CO₂-enriched gaseous stream 40. The ethanol-enriched liquid stream 30 may be further processed as desired.

The CO₂-enriched gaseous stream 40 is separated in separator 4 to obtain a H₂-enriched stream 45, which is used as a H₂ recycle stream in the RWGS reactor 2. Instead or in addition to the H₂-enriched recycle stream 45, the CO-enriched stream 20 may be separated (not shown) to obtain an H₂-enriched stream 50 (showed as a dashed line in Figure 1) and recycled to just upstream of the reactor 2.

Figure 2 shows a variation of the process of Figure 1, wherein the CO₂-containing stream 10 has been obtained from a preceding CO₂-containing stream 80 that has been subjected to a preceding bacterial fermentation in reactor 5 thereby obtaining a second ethanol-enriched liquid stream 90 and the CO₂-containing stream 10.

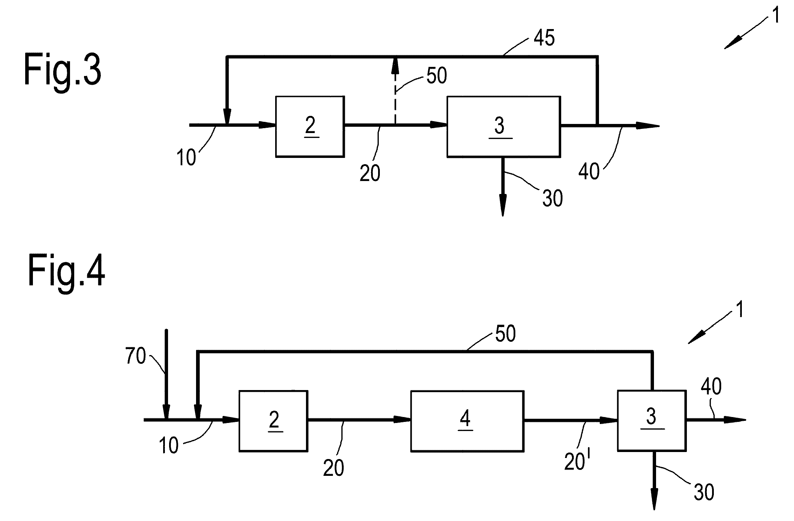

Figure 3 shows an alternative to the processes of Figs 1 and 2, wherein a CO₂ electrolysis step takes place in reactor 2. Rather than a separation (as done in separator 4 of Figs 1 and 2), in the embodiment of Figure 3, the CO₂-enriched gaseous stream 40 is split and part of it is recycled as stream 45 to just upstream of the CO₂ electrolysis reactor 2 and combined with the CO₂-containing stream 10. Preferably, the CO₂ electrolysis reactor 2 uses renewable power. Instead or in addition to the splitting, CO-enriched stream 20 may be separated (not shown) to obtain an H₂-enriched stream 50 (showed as a dashed line in Figure 3) and recycled to just upstream of the CO₂ electrolysis reactor 2.

Figure 4 shows an alternative to the processes of Figs 1 and 2, wherein a H₂-separation step occurs in separator 4 before the bacterial fermentation in reactor 3. The H₂-recycle stream 50 (obtained from the separator 4) is reused in the conversion in reactor 2, by combining it with the CO₂-containing stream 10 and H₂ make-up stream 70.

Hereinafter the invention will be further illustrated by the following non-limiting example, using the line-up of Figure 4.

### Example

Table 1 provides the conditions and the compositions of the various streams as mentioned in respect of the line-up of Fig. 4. In the bacterial fermentation, Clostridium autoethanogenum was used.

**Table 1**

| | 10 | 20 | 20' | 30 | 40 | 50 | 70 |
|---|---|---|---|---|---|---|---|
| CONDITIONS | | | | | | | |
| T [°C] | 700-900 | 40 | 40 | 40 | 40 | 40 | 40 |
| p [bara] | 52 | 52 | 52 | 52 | 52 | 52 | 52 |

| COMPONENTS [vol.%] | | | | | | | |
|---|---|---|---|---|---|---|---|
| H₂ | 56.5 | 48.2 | 8.5 | - | 8.3 | 99.0 | 100 |
| CO | 7.3 | 15.6 | 27.6 | - | 6.0 | - | - |
| CO₂ | 14.1 | 5.8 | 10.2 | - | 27.0 | - | - |
| O₂ | - | - | - | - | - | - | - |
| N₂ | 4.9 | 4.9 | 8.7 | - | 9.4 | 0.9 | - |
| Argon | 0.7 | 0.7 | 1.2 | - | 1.3 | - | - |
| H₂0 | - | - | - | 82.2 | - | - | - |
| Methane | 16.5 | 24.8 | 43.8 | - | 48.0 | 0.1 | - |
| Acetic Acid | - | - | - | 0.3 | - | - | - |
| Ethanol | - | - | - | 17.5 | - | - | - |
| TOTAL | 100 | 100 | 100 | 100 | 100 | 100 | 100 |

### Discussion

As can be seen from the Example, the present invention provides a method for producing ethanol from a CO₂-containing stream.

The person skilled in the art will readily understand that many modifications may be made without departing from the scope of the invention.

## Claims

1. A method of producing ethanol, the method at least comprising the steps of:
(a) providing a CO₂-containing stream (10);
(b) converting at least a part of the CO₂ of the CO₂-containing stream (10) provided in step (a), thereby obtaining a CO-enriched stream (20);
(c) subjecting the CO-enriched stream (20,20') obtained in step (b) to bacterial fermentation, thereby obtaining at least an ethanol-enriched liquid stream (30) and a CO₂-enriched gaseous stream (40).

2. The method according to claim 1, wherein the CO₂-containing stream (10) provided in step (a) comprises at least 5.0 vol.% CO₂, preferably at least 10.0 vol.%.

3. The method according to claim 1 or 2, wherein the CO₂-containing stream (10) provided in step (a) comprises at least 5.0 vol.% CO.

4. The method according to any of the preceding claims, wherein the CO₂-containing stream (10) provided in step (a) has been obtained from a preceding CO₂-containing stream (80) that has been subjected to a preceding bacterial fermentation step thereby obtaining a second ethanol-enriched liquid stream (90).

5. The method according to any of the preceding claims, wherein the conversion in step (b) is in the presence of hydrogen (H₂).

6. The method according to claim 5, wherein the H₂/CO₂ volume ratio of the CO₂-containing stream (10) provided in step (a) directly before it is converted in step (b) is at least 1.0, preferably at least 2.0, more preferably at least 4.0.

7. The method according to any of the preceding claims, wherein the conversion in step (b) is by a Reverse Gas Shift reaction.

8. The method according to any of the preceding claims, wherein the conversion in step (b) is performed using electrolysis.

9. The method according to any of the preceding claims, wherein the bacterial fermentation in step (c) takes place at pressure of at least 2.0 bara, preferably at least 10 bara, more preferably at least 20 bara, even more preferably at least 40 bara.

10. The method according to any of the preceding claims, wherein the CO-enriched stream (20) obtained in step (b) is separated to obtain a H₂-enriched stream (50).

11. The method according to claim 10, wherein at least a part of the H₂-enriched stream (50) is reused in the conversion in step (b).
